# EUROPÄISCHE PATENTSCHRIFT

(11) **EP 1 741 778 B1**
(45) Veröffentlichungstag und Bekanntmachung des Hinweises auf die Patenterteilung: **26.12.2007**
(21) Anmeldenummer: 05014758.6
(22) Anmeldetag: 07.07.2005
(51) Int. Cl.: C12N 13/00, C12N 15/87

(54) **Verfahren zur Behandlung geringer Volumina mit elektrischem Strom**
Procedure for the treatment of small volumes with electric current
Procédure de traitement de faibles volumes avec l'électricité électrique

(43) Veröffentlichungstag der Anmeldung: 10.01.2007
(73) Patentinhaber: Amaxa AG, 50829 Köln (DE)
(72) Erfinder: Müller-Hartmann, Herbert, 50937 Köln (DE); Habig, Michael, 4057 Basel (CH)

(56) Entgegenhaltungen:
- WO-A-20/04110371
- US-A- 5 869 326
- US-A1- 2004 014 220
- US-A1- 2005 064 596

## Beschreibung

Die Erfindung betrifft ein Verfahren zur Behandlung von biologischem Material mittels elektrischen Stroms, bei dem das biologische Material in einem geringen Volumen einer Pufferlösung mit relativ hoher lonenstärke aufgenommen und in der Pufferlösung durch einen Hochspannungsimpuls ein starkes elektrisches Feld für eine voreingestellte Dauer erzeugt wird.

Das Einbringen biologisch aktiver Moleküle, wie beispielsweise DNA, RNA oder Proteine, in lebende Zellen stellt ein wichtiges Instrument zur Untersuchung biologischer Funktionen dieser Moleküle dar. Eine bevorzugte Methode zum Einbringen von Fremdmolekülen in die Zellen ist dabei die Elektroporation, welche im Gegensatz zu chemischen Methoden nicht auf den gleichzeitigen Transport anderer biologisch aktiver Moleküle angewiesen ist. Bei der Elektroporation werden die Fremdmoleküle aus einer an die Zellen angepassten Pufferlösung oder einem Zellkulturmedium durch einen kurzzeitigen Stromfluss in die Zellen eingebracht, wobei durch die Wirkung der kurzen elektrischen Impulse die Zellmembran für die Fremdmoleküle durchlässig gemacht wird. Die Zellsuspension befindet sich dabei häufig in einer sogenannten Küvette, d.h. einem schmalen, nach oben offenen Gefäß, deren Innenraum aus jeweils zwei Paaren parallel und gegenüberliegend angeordneter Seitenwände gebildet wird. Der Innenraum dient dabei der Aufnahme der Zellsuspension, d.h. in der Regel einer wässrigen Pufferlösung oder eines Zellkulturmediums, in dem die zu behandelnden Zellen suspendiert sind. Zum Anlegen einer elektrischen Spannung weisen solche Küvetten zumeist im unteren Bereich eines Paares gegenüberliegender Seitenwände ein Elektrodenpaar auf. Bei einer elektrischen Entladung fließt zwischen den beiden Elektroden ein elektrischer Strom durch die Zellsuspension, der einen Transport der Nukleinsäuren oder anderer Moleküle in die Zellen bewirkt oder je nach den gewählten Bedingungen zur Zellfusion führt.

Durch das kurzzeitige Anlegen eines starken elektrischen Feldes, d.h. eines kurzen Pulses mit hoher Stromdichte, können Zellen, Zellderivate, subzelluläre Partikel und/oder Vesikel auch fusioniert werden. Bei dieser sogenannten Elektrofusion werden die Zellen beispielsweise zunächst durch ein inhomogenes elektrisches Wechselfeld in engen Membrankontakt gebracht. Durch das anschließende Anlegen eines elektrischen Feldpulses kommt es dann zur Interaktion von Membranteilen, die schließlich zur Fusion führt. Für die Elektrofusion können dabei vergleichbare apparative Vorrichtungen verwendet werden, wie für die Elektroporation.

Bei der Elektroporation gelangen die biologisch aktiven Moleküle durch die kurzzeitig entstehenden "Poren" in der Zellmembran zunächst in das Zytoplasma, in dem sie ggf. bereits ihre zu untersuchende Funktion ausüben können, und daraufhin unter bestimmten Bedingungen auch in den Zellkern. Insbesondere bei Anwendungen, bei denen die biologisch aktiven Moleküle ihre zu untersuchende Funktion nur im Zellkern ausüben können, beispielsweise wenn die Expression eines Gens untersucht werden soll, und Zellen ohne oder mit nur geringer Teilungsaktivität verwendet werden, beispielsweise primäre Zellen, ist es von Vorteil, wenn die biologisch aktiven Moleküle direkt in den Zellkern transportiert werden.

Aus dem in der WO 02/00871 A2 beschriebenen Elektroporationsverfahren ist bekannt, dass in solchen Fällen in der Pufferlösung durch einen Hochspannungsimpuls ein starkes elektrisches Feld mit einer Feldstärke von mindestens 2 kV/cm für eine voreingestellte Dauer von mindestens 10 µs erzeugt werden muss, um hohe Transfektionseffizienzen zu erzielen.

Aus der WO 02/086134 A2 ist ebenfalls ein Verfahren zur. Behandlung von biologischem Material mittels hoher elektrischer Ströme bekannt, bei dem das biologische Material in einer Pufferlösung mit einer lonenstärke von mindestens 200 mmol/l aufgenommen wird, um bei hoher Transfektionseffizienz eine geringe Mortalitätsrate der Zellen zu gewährleisten.

Vor allem bei biochemischen und pharmazeutischen Anwendungen, bei denen eine Vielzahl von Reaktionsansätzen gleichzeitig und in möglichst kurzer Zeit getestet werden muss, insbesondere bei HT-Analysen (HT = high throughput), ist es erforderlich, eine möglichst große Anzahl von Reaktionsräumen, beispielsweise 96 oder 384, zur Verfügung zu stellen. Bei den dabei verwendeten Reaktionsgefäßen spricht man in der Regel von Mehrlochplatten, Mikrotiterplatten oder Multiwells. Die einzelnen Reaktionsräume ("wells") dieser Gefäße sind relativ klein und können daher nur geringe Volumina aufnehmen. Darüber hinaus ist es ohnehin häufig von Vorteil geringere Probenvolumina einzusetzen, um Puffer und Zellmaterial zu sparen. Insbesondere bei wertvollem Zellmaterial, beispielsweise primären Zellen, stehen dabei in der Regel nur geringe Mengen an Zellen zur Verfügung. Es ist also häufig wünschenswert oder notwendig, mit geringen Probenvolumina zu arbeiten.

Bei der Erzeugung von starken elektrischen Feldern in Flüssigkeiten lässt sich als Nebeneffekt elektrische Hydrolyse nicht ausschließen. Im mildesten Falle macht sich die Elektrolyse durch die Bildung von Gasblasen an den Elektrodenoberflächen bemerkbar, was zur Schaumbildung führt. Im extremen Falle kommt es zu einer explosionsartigen Gasbildung, die durch den resultierenden Verdrängungseffekt zum Austreiben der Proben aus dem Bereich zwischen den Elektroden führt (im Folgenden als "Spritzen" bezeichnet). Letzteres führt in der Regel zum Verlust der Proben oder zumindest dazu, dass die Probe nicht bis zum Abschluss der vorgesehenen Zeit im elektrischen Feld verweilt. Somit beeinträchtigt das Spritzen einer Probe das Ergebnis eines Versuch oder einer Probenprozessierung qualitativ und/oder quantitativ und wirkt sich ferner negativ auf die Reproduzierbarkeit der Ergebnisse aus. Folglich stellt die Elektrolyse bei den verschiedenen Anwendungen, bei denen eine Behandlung von biologischen Zellen im elektrischen Feld notwendig ist, insbesondere bei der Elektroporation, einen unerwünschten Nebeneffekt dar.

Theoretisch könnte man die Spritzwahrscheinlichkeit durch eine Absenkung der Leitfähigkeit erreichen. Höhere Zellen, die mit keiner festen Zellwand ausgestattet sind, können aber in der Regel nur in Lösungen mit einer gewissen Osmolarität überdauern. Meist sind unter den osmotisch wirksamen gelösten Substanzen auch Elektrolyte, die in einer mehr oder minder hohen Leitfähigkeit der Lösung resultieren. Beispielsweise ist zur Durchführung einer Elektroporation in der Regel der Einsatz von Ionen in der Zellsuspension notwendig und, wie in der WO 02/086134 A2 dargelegt, auch vorteilhaft. Folglich ist man aus praktischen Gründen darin begrenzt, die Spritzwahrscheinlichkeit durch Absenkung der Leitfähigkeit zu reduzieren. Somit ist hier in allen Fällen von Elektrolyse mehr oder minder großen Umfanges auszugehen.

Das Ereignis des Spritzens ist dabei ein stochastisches Ereignis. Dies bedeutet, dass man das Ereignis nur durch Wahrscheinlichkeiten beschreiben kann. In Abhängigkeit von den vorgegebenen Bedingungen kann die Häufigkeit des unerwünschten Spritzens beispielsweise unter 5 % oder aber über 95 % liegen. Die Wahrscheinlichkeit des Spritzens ist dabei besonders hoch, wenn geringe Volumina bei hoher Stromdichte eingesetzt werden. Um einen Prozess zur Produktreife zu entwickeln, stellt sich daher das Problem, diese Wahrscheinlichkeit bei der Anwendung entsprechender Verfahren durch den Kunden auf beispielsweise unter 1 % zu senken.

Es ist folglich Aufgabe der Erfindung, ein Verfahren der eingangs genannten Art zu schaffen, bei dem die Häufigkeit des unerwünschten Austreibens einer Probe aus dem Bereich zwischen den Elektroden deutlich reduziert ist.

Erfindungsgemäß wird die Aufgabe durch ein Verfahren gelöst, bei dem das biologische Material in höchstens 50 µl einer Pufferlösung mit einer lonenstärke von mindestens 100 mmol/l aufgenommen und in der Pufferlösung durch zumindest einen Spannungsimpuls ein elektrisches Feld mit einer Feldstärke von mindestens 1 kV/cm für eine voreingestellte Dauer von mindestens 10 µs erzeugt wird, wobei der Spannungsimpuls mindestens einmal für eine Zeitspanne von mindestens 100 µs unterbrochen und anschließend wieder fortgesetzt wird. Die voreingestellte Dauer des Spannungsimpulses entspricht dabei der reinen Dauer des Impulses ohne Unterbrechungen, d. h. der gesamten Netto-Zeit, in der tatsächlich Strom fließt. Durch die dazu kommenden Zeitspannen, in denen der Spannungsimpuls unterbrochen wird, erhöht sich also die Gesamtdauer des Impulses entsprechend, so dass der Spannungsimpuls von seiner Auslösung bis zum Ende, d. h. bis zum Erreichen der voreingestellten Dauer, tatsächlich länger ist als die voreingestellte Dauer. Der Spannungsimpuls wird beim erfindungsgemäßen Verfahren nach einer vorbestimmten Zeit für die vorgegebene Zeitspanne unterbrochen und anschließend wieder fortgesetzt, wobei dieser Vorgang so oft wiederholt wird, bis die voreingestellte Dauer des Spannungsimpulses erreicht ist. Der Spannungsimpuls muss dabei so oft unterbrochen werden, dass die Gasentwicklung in der Pufferlösung ausreichend verringert wird, um ein Austreiben der Probe aus dem Bereich zwischen den Elektroden zu verhindern. Die Zeitspannen der einzelnen Unterbrechungen müssen ferner ausreichend lang sein, um ein Austreiben der Probe aus dem Bereich zwischen den Elektroden zu verhindern. Die Häufigkeit des Spritzens lässt sich bei ansonsten vorgegebenen Bedingungen folglich dadurch deutlich reduzieren, dass der Spannungsimpuls unterbrochen wird, wobei die Effizienz des Verfahrens, insbesondere die Transfektionseffizienz, erhalten bleibt.

Die Häufigkeit des unerwünschten Spritzens hängt einerseits, beispielsweise bei der Elektroporation, von der Stromdichte (Feldstärke mal spezifische Leitfähigkeit) und dem Zeitintervall, während dem das elektrische Feld anliegt, positiv ab. Stromdichte und Zeitintervall stehen dabei mit der Menge des durch Elektrolyse erzeugten Gases pro Probenvolumen in direktem Zusammenhang. Die Feldstärke und das Zeitintervall geben die Bedingungen vor, unter denen beispielsweise hohe Transfektionseffizienzen oder ein direkter Transport der Moleküle in den Zellkern erreicht werden können. Eine Verringerung eines dieser beiden Parameter weg von den optimalen zelltyp-spezifischen Bedingungen führt in aller Regel zu einem qualitativen und/oder quantitativen Abfall der Versuchsergebnisse. Somit kommt hier eine Verminderung eines dieser Parameter nicht oder nur begrenzt in Frage. Auf der anderen Seite hängt die Spritzwahrscheinlichkeit vom Volumen der Probe (Masse) negativ ab. Diese Abhängigkeit ergibt sich aus der größeren Trägheit eines größeren Volumens. Größere Volumina bleiben innerhalb des kurzen Zeitfensters eher im Küvettenspalt zurück. Somit kommt es bei kleinen Volumina bei ansonsten gleichen Bedingungen besonders häufig zum Spritzen. Das Spritzproblem spielt also bei Verfahren im HT-Bereich eine besondere Rolle. Allerdings lässt sich für bestimmte Anwendungen, insbesondere aus dem Hoch-Durchsatz-Bereich, das Volumen einer Probe nicht steigern. Zum einen ist eine größere Anzahl von Proben bei zunehmendem Volumen nicht oder nur mit erhöhtem Aufwand durch automatisierte Liquid-Handling-Systeme zu handhaben und zum anderen ist hier aus Kostengründen die Menge des zur Verfügung stehenden Zellmaterials (und u. U. der Reagenzien) oft begrenzt. Darüber hinaus kann die Verwendung eines großen Volumens auch aus anderen Gründen nachteilig sein. Eine Absenkung der Zellkonzentration führt ab einem gewissen Umfang zu deutlich schlechteren Ergebnissen, beispielsweise bei der Elektroporation bezüglich der Überlebensrate der Zellen oder bei der Elektrofusion bezüglich der Effizienz. Somit ist insbesondere bei HT-Verfahren die Vergrößerung des Volumens keine Alternative. Folglich stellt die durch das erfindungsgemäße Verfahren zur Verfügung gestellte Lösung eine effektive und sichere Möglichkeit dar, die Häufigkeit des Spritzens unter ansonsten vorgegebenen Bedingungen deutlich zu reduzieren.

In vorteilhafter Ausgestaltung der Erfindung ist vorgesehen, dass der Spannungsimpuls zwei- bis zehnmal unterbrochen wird. Dabei hängt die Anzahl der Unterbrechungen von den vorgegebenen Bedingungen ab, insbesondere der Stromdichte, der Dauer des Impulses und dem zur Verfügung stehenden Volumen. Die optimale Anzahl der Unterbrechungen muss ferner häufig für die jeweilige Anwendung und/oder den verwendeten Zelltyp empirisch ermittelt werden.

Erfindungsgemäß kann für zumindest eine Unterbrechung eine Zeitspanne von 200 µs bis 2 ms, vorzugsweise 300 µs, 400 µs, 500 µs, 600 µs, 700 µs, 800 µs, 900 µs, 1 ms oder 1,5 ms, vorgegeben werden. Solange die Unterbrechungsintervalle eine gewisse Länge nicht überschreiten, kann das Spritzen der Probe verhindert werden, ohne dass dies negative Auswirkungen auf die Qualität der Verfahrensergebnisse hat. Die optimale Zeitspanne für die Unterbrechung bzw. die Unterbrechungen muss dabei in der Regel für die jeweilige Anwendung und/oder den verwendeten Zelltyp empirisch ermittelt werden. Die Zeitspanne, die für die Reduzierung der Wahrscheinlichkeit des Spritzens erforderlich ist, hängt ferner insbesondere von den vorgegebenen Bedingungen, d. h. der Stromdichte, der Dauer des Impulses und dem zur Verfügung stehenden Volumen, ab.

Das Volumen der Pufferlösung mit dem biologischen Material kann beispielsweise insgesamt zwischen 1 und 50 µl, vorzugsweise 10 bis 40 µl, besonders bevorzugt 15 bis 25 µl, insbesondere 10 bis 20 µl, liegen. Dabei ergibt sich das eingesetzte Volumen in der Regel aus der Verfügbarkeit des biologischen Materials und/oder den verfahrenstechnischen Gegebenheiten.

In vorteilhafter Ausgestaltung der Erfindung ist vorgesehen, dass der Spannungsimpuls ein elektrisches Feld mit einer Feldstärke von maximal 10 kV/cm, vorzugsweise 1 bis 8 kV/cm, besonders bevorzugt 2 bis 6 kV/cm, insbesondere 2 bis 4 kV/cm, erzeugt. Solche Hochspannungsimpulse eignen sich besonders für die Elektroporation von eukaryontischen Zellen, insbesondere das Einbringen von Nukleinsäuren in den Zellkern.

In weiterer vorteilhafter Ausgestaltung der Erfindung ist vorgesehen, dass der Spannungsimpuls eine voreingestellte Dauer von maximal 5 ms, vorzugsweise 20 µs bis 2 ms, besonders bevorzugt 100 bis 1000 µs, insbesondere 100 bis 600 µs, aufweist. Die voreingestellte Dauer ist dabei die vorgegebene Länge des Spannungsimpulses ohne Unterbrechungen, d. h. die Zeitdauer, in der tatsächlich Spannung anliegt bzw. Strom fließt. Die voreingestellte Dauer ist in der Regel ein empirisch ermittelter Wert, der für die jeweilige Anwendung und/oder den verwendeten Zelltyp optimal ist. Sollten bei besonderen Anwendungen aufgrund der beim erfindungsgemäßen Verfahren vorgesehenen Unterbrechungen des Spannungsimpulses geringfügig verringerte Effizienzen zu erwarten sein, so können dieses in bestimmten Fällen beispielsweise dadurch wieder verbessert werden, dass die voreingestellte Dauer in gewissen Grenzen über den empirisch ermittelten Wert hinaus verlängert wird. Auf diese Weise könnten in Einzelfällen mögliche negative Auswirkungen des Unterbrechens des Spannungsimpulses kompensiert werden.

In vorteilhafter Ausgestaltung der Erfindung ist ferner vorgesehen, dass der Spannungsimpuls annähernd nach einem Spannungsintervall von 5 µs, 10 µs, 20 µs, 30 µs, 40 µs, 50 µs, 60 µs, 100 µs oder 200 µs unterbrochen wird. Hierbei kann es sich um das erste Spannungsintervall oder um eines oder mehrere der nachfolgenden Spannungsintervalle handeln. Ein Spannungsintervall ist dabei ein Zeitraum, in dem Spannung anliegt bzw. ein elektrisches Feld in der Pufferlösung erzeugt wird und auf das ein Unterbrechungsintervall folgt oder dem ein Unterbrechungsintervall vorhergeht. Durch das Verkürzen eines oder mehrerer Spannungsintervalle kann unter bestimmten Bedingungen die Spritzwahrscheinlichkeit weiter reduziert bzw. minimiert werden.

Das elektrische Feld kann erfindungsgemäß zwischen zwei Elektroden erzeugt werden, wobei der Abstand zwischen den beiden Elektroden in besonders vorteilhafter Ausgestaltung der Erfindung 0,5 bis 5 mm, vorzugsweise 1 bis 4 mm, insbesondere 1,5 bis 2 mm, betragen kann. In jedem Fall muss der Abstand zwischen den Elektroden in Abhängigkeit von der Geometrie des verwendeten Reaktionsbehältnisses derart dimensioniert sein, dass das zur Verfügung stehende Volumen der Pufferlösung den Raum zwischen den Elektroden ausreichend benetzen kann.

In vorteilhafter Ausgestaltung der Erfindung ist ferner vorgesehen, dass die Behandlung des biologischen Materials in einem Reaktionsbehältnis durchgeführt wird, das einen im Wesentlichen quadratischen, rechteckigen oder runden Querschnitt hat. Dabei kann das Reaktionsbehältnis einen im Wesentlichen rechteckigen Reaktionsraum aufweisen, der seitlich durch zwei planparallel angeordnete Elektroden begrenzt ist.

Die Erfindung wird im Weiteren anhand der Figuren beispielhaft näher erläutert.

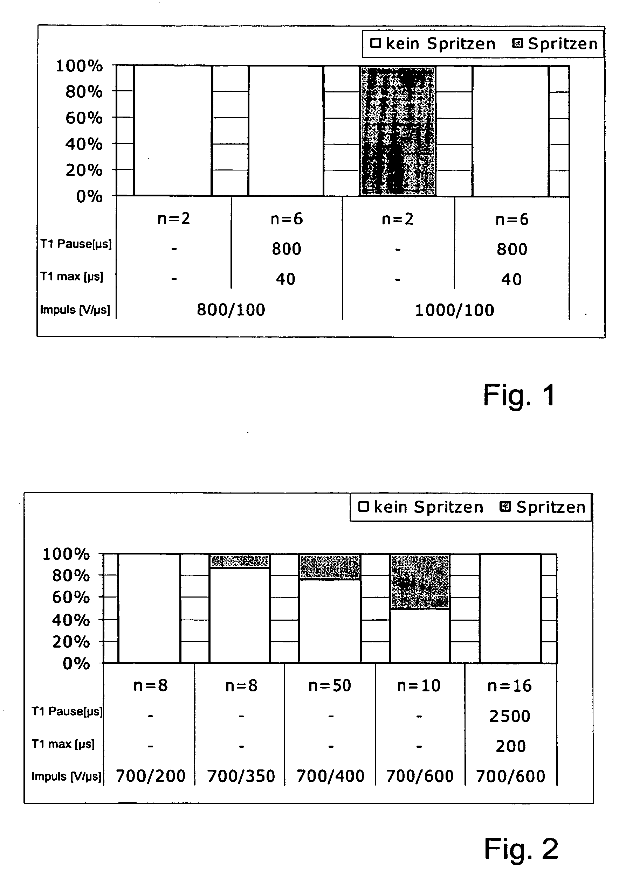

Es zeigt
- Figur 1: ein Balkendiagramm der Spritzhäufigkeit in Abhängigkeit von der Spannung, high-throughput Nucleofector® (HT-beta, Fa. amaxa GmbH), Volumen der Zellsuspension: 20 µl, Spaltbreite der Küvette: 1,5 mm, lonenstärke der Pufferlösung: 203 mmol/l (Leitfähigkeit: 11,3 mS/cm), die schwarz hinterlegte Fläche zeigt die Häufigkeit des Spritzens der einzelnen Proben an, n = Anzahl der Proben,
- Figur 2: ein Balkendiagramm der Spritzhäufigkeit in Abhängigkeit von der Impulsdauer, high-throughput Nucleofector® (HT-beta, Fa. amaxa GmbH), Volumen der Zellsuspension: 20 µl, Spaltbreite der Küvette: 1,5 mm, lonenstärke der Pufferlösung: 129 mmol/l (Leitfähigkeit: 7,2 mS/cm), die schwarz hinterlegte Fläche zeigt die Häufigkeit des Spritzens der einzelnen Proben an, n = Anzahl der Proben,

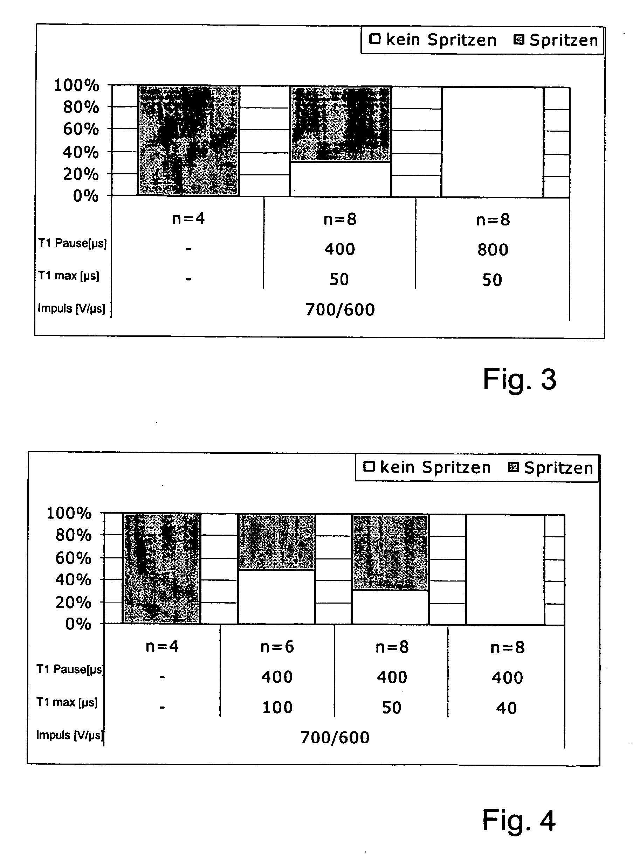

- Figur 3: ein Balkendiagramm der Spritzhäufigkeit in Abhängigkeit von der Zeitspanne der Unterbrechung des Spannungsimpulses, high-throughput Nucleofector® (HT-beta, Fa. amaxa GmbH), Volumen der Zellsuspension: 20 µl, Spaltbreite der Küvette: 1,5 mm, lonenstärke der Pufferlösung: 203 mmol/l (Leitfähigkeit: 11,3 mS/cm), die schwarz hinterlegte Fläche zeigt die Häufigkeit des Spritzens der einzelnen Proben an, n = Anzahl der Proben,
- Figur 4: ein Balkendiagramm der Spritzhäufigkeit in Abhängigkeit von der maximalen Dauer eines ununterbrochenen Spannungsintervalls, high-throughput Nucleofector® (HT-beta, Fa. amaxa GmbH), Volumen der Zellsuspension: 20 µl, Spaltbreite der Küvette: 1,5 mm, lonenstärke der Pufferlösung: 203 mmol/l (Leitfähigkeit: 11,3 mS/cm), die schwarz hinterlegte Fläche zeigt die Häufigkeit des Spritzens der einzelnen Proben an, n = Anzahl der Proben,

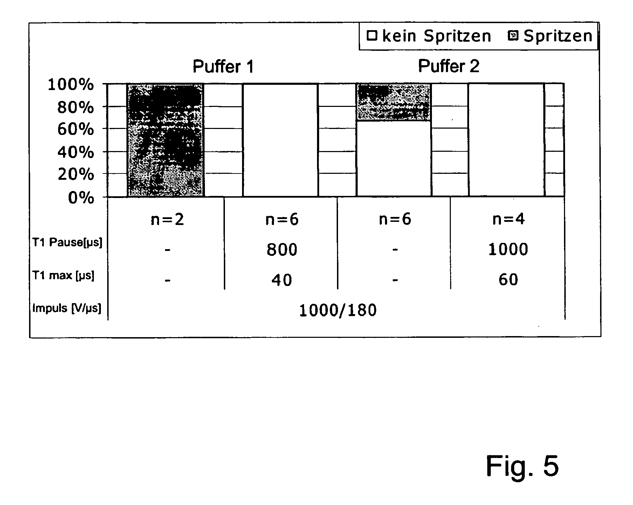

- Figur 5: ein Balkendiagramm der Spritzhäufigkeit in Abhängigkeit von der Leitfähigkeit bzw. lonenstärke der Pufferlösung, high-throughput Nucleofector® (HT-beta, Fa. amaxa GmbH), Volumen der Zellsuspension: 20 µl, Spaltbreite der Küvette: 1,5 mm, lonenstärke der Pufferlösung:), die schwarz hinterlegte Fläche zeigt die Häufigkeit des Spritzens der einzelnen Proben an, n = Anzahl der Proben, Puffer 1: lonenstärke 203 mmol/l und Leitfähigkeit 11,3 mS/cm, Puffer 2: lonenstärke 129 mmol/l und Leitfähigkeit 7,2 mS/cm,

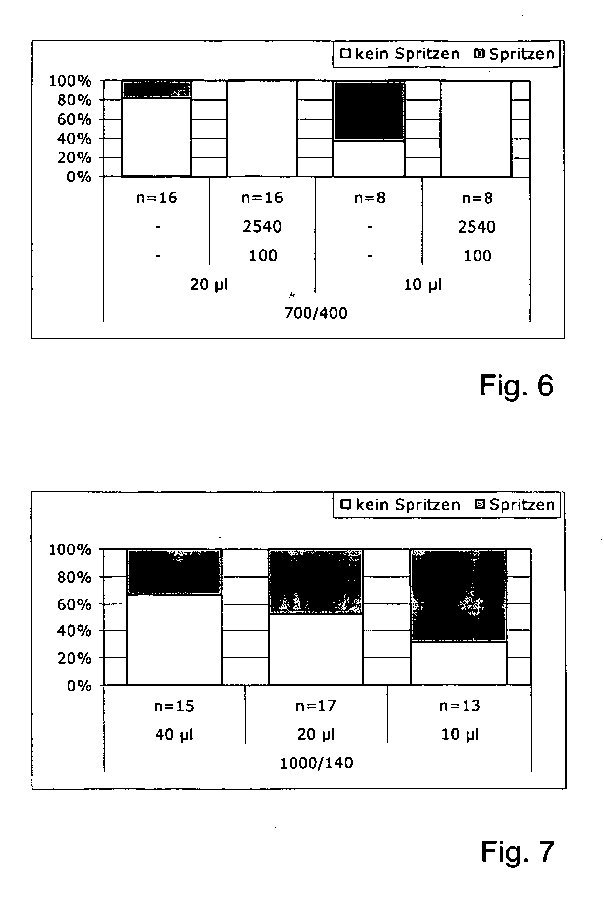

- Figur 6: ein Balkendiagramm der Spritzhäufigkeit in Abhängigkeit vom Volumen der Zellsuspension, high-throughput Nucleofector® (HT-beta, Fa. amaxa GmbH), Spaltbreite der Küvette: 1,5 mm, lonenstärke der Pufferlösung: 129 mmol/l (Leitfähigkeit: 7,2 mS/cm), die schwarz hinterlegte Fläche zeigt das Spritzen der einzelnen Proben an, n = Anzahl der Proben,
- Figur 7: ein Balkendiagramm der Spritzhäufigkeit in Abhängigkeit vom Volumen der Zellsuspension, high-throughput Nucleofector® (HT-beta, Fa. amaxa GmbH), Spaltbreite der Küvette: 1,5 mm, lonenstärke der Pufferlösung: 203 mmol/l (Leitfähigkeit: 11,3 mS/cm), die schwarz hinterlegte Fläche zeigt die Häufigkeit des Spritzens der einzelnen Proben an, n = Anzahl der Proben,

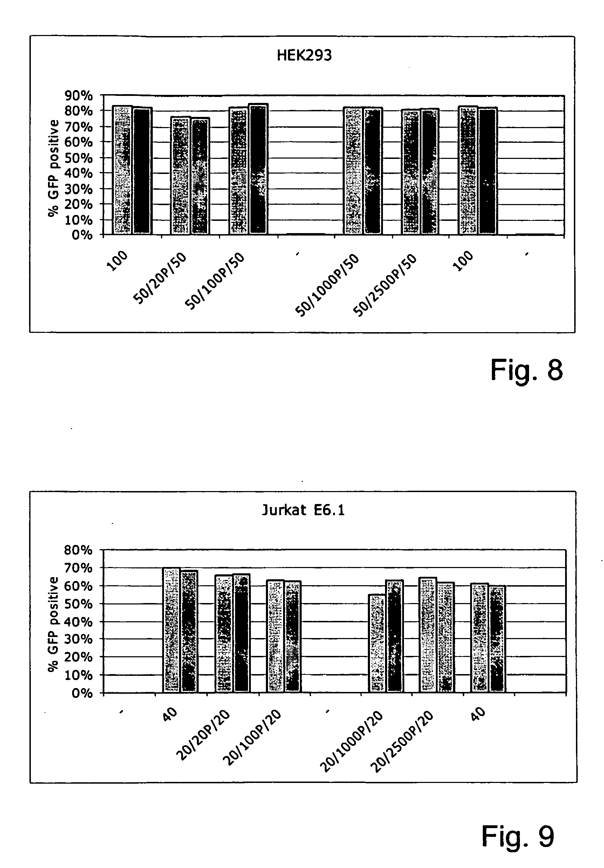

- Figur 8: ein Balkendiagramm der Transfektionseffizienzen in Abhängigkeit von der Zeitspanne der Unterbrechung des Spannungsimpulses, high-throughput Nucleofector® (HT-beta, Fa. amaxa GmbH), Spaltbreite der Küvette: 1,5 mm, lonenstärke der Pufferlösung: 203 mmol/l (Leitfähigkeit: 11,3 mS/cm), zur Ermittlung der Transfektionseffizienz wurden je 2x10⁵ HEK293-Zellen in 20 µl Pufferlösung aufgenommen, mit 0,1 µg pEGFP-C1 (Invitrogen) versetzt und einem Feld von 4 kV/cm ausgesetzt, anschließend wurden die Zellen in Minimum Essential Medium Eagle (ATCC) mit 100 µg/ml Streptomycin, 100 U/ml Penicillin und 10% Horse Serum (ATCC) aufgenommen und für 24 Stunden bei 37°C und 5% CO₂ in einem befeuchteten Kulturschrank kultiviert, abschließend wurden die Proben mittels Durchflusszytometrie (FACSCalibur, Becton Dickinson) auf GFP-Expression untersucht, dargestellt sind jeweils Doppelwerte der prozentualen Anteile der GFP-exprimierenden Zellen, die Beschriftung der Balken bezieht sich auf die Unterbrechung der Feldexposition: alle Angaben sind in µs, die Zahlen am Anfang und am Ende stehen für die Dauer der ununterbrochenen Spannungsintervalle, die Zahlen zwischen den Querstrichen für die Zeitspanne der dazwischen liegenden Unterbrechung (P = Pause), und
- Figur 9: ein Balkendiagramm der Transfektionseffizienzen in Abhängigkeit von der Zeitspanne der Unterbrechung des Spannungsimpulses, high-throughput Nucleofector® (HT-beta, Fa. amaxa GmbH), Spaltbreite der Küvette: 1,5 mm, lonenstärke der Pufferlösung: 203 mmol/l (Leitfähigkeit: 11,3 mS/cm), zur Ermittlung der Transfektionseffizienz wurden je 2x10⁵ Jurkat E6.1-Zellen in 20 µl Pufferlösung aufgenommen, mit 1 µg pmaxGFP (amaxa) versetzt und einem Feld von 1,25 kV/cm ausgesetzt, anschließend wurden die Zellen in RPMI-1640 Medium (ATCC) mit 100 µg/ml Streptomycin, 100 U/ml Penicillin und 10% FCS (ATCC) aufgenommen und für 24 Stunden bei 37°C und 5% CO₂ in einem befeuchteten Kulturschrank kultiviert, abschließend wurden die Proben mittels Durchflusszytometrie (FACSCalibur, Becton Dickinson) auf GFP-Expression untersucht, dargestellt sind jeweils Doppelwerte der prozentualen Anteile der GFP-exprimierenden Zellen, die Beschriftung der Balken bezieht sich auf die Unterbrechung der Feldexposition: alle Angaben sind in µs, die Zahlen am Anfang und am Ende stehen für die Dauer der ununterbrochenen Spannungsintervalle, die Zahlen zwischen den Querstrichen für die Zeitspanne der dazwischen liegenden Unterbrechung (P = Pause).

Figur 1 zeigt ein Balkendiagramm der Spritzhäufigkeit in Abhängigkeit von der Spannung in Form eines Vergleichs zweier unterschiedlicher Spannungsimpulse, die jeweils mit und ohne Unterbrechung ausgeführt wurden. Der erste Spannungsimpuls hat eine voreingestellte Dauer von 100 µs bei einer außen angelegten Spannung von 800 V, wobei sich aus der Spannung rechnerisch unter Einbeziehung des Elektrodenwiderstands eine Feldstärke von ca. 4 kV/cm ergibt. Der zweite Spannungsimpuls hat ebenfalls eine voreingestellte Dauer von 100 µs, allerdings bei einer angelegten Spannung von 1000 V, wobei sich aus der Spannung rechnerisch eine Feldstärke von ca. 5 kV/cm ergibt. Beide Spannungsimpulse wurden jeweils nach 40 µs (T1 max) zweimal unterbrochen, d.h. der Impuls wurde nach 40 µs unterbrochen, dann fortgesetzt, nach weiteren 40 µs erneut unterbrochen und schließlich mit einem Spannungsintervall von 20 µs vollendet, so dass insgesamt die voreingestellte Dauer von 100 µs erreicht wurde. Die Zeitspanne der Unterbrechungen betrug jeweils 800 µs (T1 Pause). Insgesamt setzt sich jeder Spannungsimpuls also aus 3 Spannungsintervallen und 2 Unterbrechungsintervallen zusammen, wobei sich die Gesamtdauer des Impulses auf insgesamt 1.700 µs addiert. Es wird hier deutlich, dass bei der Anwendung des Spannungsimpulses mit der geringeren Spannung bzw. Feldstärke unter den vorgegebenen Bedingungen kein Austreiben der Proben erfolgt, d.h. es spritzt hier nicht. Dagegen kommt es bei dem Spannungsimpuls mit der höheren Spannung bzw. Feldstärke zum Austreiben der Proben (dritter Balken). Dieses unerwünschte Spritzen kann durch die zweimalige Unterbrechung des Spannungsimpulses verhindert werden (letzter Balken). Es zeigt sich also einerseits, dass die Wahrscheinlichkeit des Spritzens bei ansonsten konstanten Bedingungen mit steigender Feldstärke höher wird, und andererseits, dass ein Austreiben der Probe aus dem Reaktionsgefäß auch bei sehr hohen Feldstärken durch das Unterbrechen des Spannungsimpulses verhindert werden kann. Zumindest wird durch das Unterbrechen des Impulses die Wahrscheinlichkeit des Spritzens signifikant reduziert.

Figur 2 zeigt ein Balkendiagramm der Spritzhäufigkeit in Abhängigkeit von der Impulsdauer, wobei Spannungsimpulse mit einer Stärke von jeweils 700 V (3,5 kV/cm Feldstärke) mit ansteigender voreingestellter Dauer von 200, 350, 400 und 600 µs angewendet wurden. Dabei zeigte sich, dass die prozentuale Häufigkeit des Spritzens mit steigender Impulsdauer ansteigt. Durch das erfindungsgemäße Unterbrechen des Spannungsimpulses mit der der längsten voreingestellten Dauer von 600 µs konnte das Spritzen der Proben aber effektiv verhindert werden. Die Spannungsimpulse wurden zu diesem Zweck jeweils nach 200 µs (T1 max) zweimal unterbrochen, d.h. der Impuls wurde nach 200 µs unterbrochen, dann fortgesetzt, nach weiteren 200 µs erneut unterbrochen und schließlich mit einem Spannungsintervall von erneut 200 µs vollendet, so dass insgesamt die voreingestellte Dauer von 600 µs erreicht wurde. Die Zeitspanne der Unterbrechungen betrug jeweils 2,5 ms (T1 Pause). Insgesamt setzt sich hier jeder Spannungsimpuls also aus 3 Spannungsintervallen und 2 Unterbrechungsintervallen zusammen, wobei sich die Gesamtdauer des Impulses auf insgesamt 5,6 ms addiert. Es wird hier deutlich, dass es bei relativ langer voreingestellter Dauer des Spannungsimpulses mit hoher Wahrscheinlichkeit zum Austreiben der Proben kommt (vierter Balken). Dieses unerwünschte Spritzen kann durch die zweimalige Unterbrechung des Spannungsimpulses verhindert werden (letzter Balken). Es zeigt sich folglich einerseits, dass die Wahrscheinlichkeit des Spritzens bei ansonsten konstanten Bedingungen mit steigender Impulsdauer höher wird, und andererseits, dass ein Austreiben der Probe aus dem Reaktionsgefäß auch bei relativ langer voreingestellter Dauer durch das Unterbrechen des Spannungsimpulses verhindert werden kann.

Figur 3 zeigt ein Balkendiagramm der Spritzhäufigkeit in Abhängigkeit von der Zeitspanne der Unterbrechung des Spannungsimpulses. An diesem Beispiel wird deutlich, dass zumindest bei bestimmten Anwendungen bzw. Bedingungen durch die Verlängerung der Zeitspanne der Unterbrechung(en) die Wahrscheinlichkeit des Austreibens der Probe aus dem Reaktionsgefäß zusätzlich verringert werden kann. Ein Spannungsimpuls mit einer Spannung von 700 V (3,5 kV/cm Feldstärke) und einer voreingestellten Dauer von 600 µs wurde ununterbrochen (erster Balken), 11 x für je 400 µs unterbrochen (zweiter Balken) oder 11 x für je 800 µs unterbrochen (dritter Balken) angewendet. Die unterbrochenen Impulse setzen sich also aus 12 Spannungsintervallen von je 50 µs Länge (T1 max) und 11 Unterbrechungsintervallen von je 400 µs oder 800 µs (T1 Pause) zusammen. Wird der Spannungsimpuls unter diesen Bedingungen nicht erfindungsgemäß unterbrochen, so spritzt es bei jedem Versuch bzw. jeder Probe. Während es bei einer Zeitspanne der Unterbrechungen von 400 µs noch bei etwa 70 % der Proben spritzt, kann das Spritzen dagegen durch die Verdoppelung der Zeitspanne der Unterbrechungen unter diesen Bedingungen vollständig verhindert werden. Offensichtlich "beruhigen" sich die Verhältnisse in der Pufferlösung mit zunehmender Länge der Unterbrechung(en), so dass es während des der Unterbrechung nachfolgenden Spannungsintervalls nicht mehr zur Gasbildung in der Probe kommt.

Figur 4 zeigt ein Balkendiagramm der Spritzhäufigkeit in Abhängigkeit von der maximalen Dauer eines ununterbrochenen Spannungsintervalls. Dieser Versuch wurde praktisch unter den gleichen Bedingungen wie der Versuch gemäß Figur 3 durchgeführt, mit dem Unterschied, dass die Zeitspanne der Unterbrechungen (T1 Pause) konstant bei 400 µs gehalten wurde, während die maximale Länge der Spannungsintervalle (T1 max), d.h. der Zeiträume, in denen ein elektrisches Feld erzeugt wird, variiert wurde. Es wird beim Vergleich der Versuche mit T1 max = 100 µs und T1 max = 40 µs deutlich, dass eine Verkürzung des Spannungsintervalls, d.h. der Zeit, bis zu der der Spannungsimpuls unterbrochen wird, zu einer Verringerung der Spritzwahrscheinlichkeit führt. Dabei deutet im vorliegenden Ausführungsbeispiel einiges darauf hin, dass hier ein Schwellenwert zwischen 40 und 50 µs existiert, d.h. in diesem Fall keine proportionale Abhängigkeit der Spritzhäufigkeit von T1 max besteht.

Figur 5 zeigt ein Balkendiagramm der Spritzhäufigkeit in Abhängigkeit von der Leitfähigkeit bzw. lonenstärke der Pufferlösung. Bei Verwendung eines Puffers mit höherer lonenstärke (Puffer 1: lonenstärke 203 mmol/l und Leitfähigkeit 11,3 mS/cm) ist die Gefahr des Spritzens wesentlich größer als bei der Verwendung eines Puffers mit geringerer lonenstärke (Puffer 2: lonenstärke 129 mmol/l und Leitfähigkeit 7,2 mS/cm). In beiden dargestellten Beispielen kann das Spritzen jedoch durch das erfindungsgemäße Unterbrechen des Spannungsimpulses verhindert werden.

Die Figuren 6 und 7 zeigen jeweils ein Balkendiagramm der Spritzhäufigkeit in Abhängigkeit vom Volumen der Zellsuspension. Aus beiden Abbildungen wird deutlich, dass die Wahrscheinlichkeit des Spritzens umso höher ist, je geringer das Volumen der Zellsuspension bzw. Pufferlösung ist. Das Beispiel gemäß Figur 6 zeigt in diesem Zusammenhang, dass die Spritzwahrscheinlichkeit auch bei Anwendungen, bei denen nur sehr geringe Volumina eingesetzt werden können, praktisch auf Null reduziert werden kann.

Die Figuren 8 und 9 zeigen jeweils ein Balkendiagramm der Transfektionseffizienzen in Abhängigkeit von der Zeitspanne der Unterbrechung des Spannungsimpulses. Die Transfektionseffizienzen werden durch das erfindungsgemäße Unterbrechen der Spannungsimpulse überraschender Weise nicht negativ beeinflusst. Die Effizienz des Verfahrens bewegt sich immer auf einem in etwa gleich bleibenden hohen Niveau, unabhängig davon, ob der Spannungsimpuls unterbrochen wird (Doppelbalken 2 bis 5) oder nicht (Doppelbalken 1 und 6).

Insgesamt zeigt sich also, dass bei ansonsten vorgegebenen Bedingungen (Feldstärke bzw. Stromdichte, voreingestellte Dauer des Spannungsimpulses, Volumen der Pufferlösung) eine bestimmte ununterbrochene Länge eines Spannungsintervalls nicht überschritten werden darf, um Spritzereignisse zu vermeiden bzw. die Wahrscheinlichkeit des Austreibens der Probe aus dem Reaktionsgefäß ausreichend zu vermindern. Somit lässt sich das Spritzproblem bei ansonsten vorgegebenen Bedingungen lösen, indem der Impuls unterbrochen bzw. eine kritische ununterbrochene Länge eines Spannungsintervalls nicht überschritten wird. Dabei führt die Unterbrechung der Spannungsimpulse zu einer deutlichen Reduzierung der Spritzwahrscheinlichkeit, ohne dass die Qualität des Verfahrens, hier insbesondere die Transfektionseffizienz, beeinträchtigt wird.

## Patentansprüche

1. Verfahren zur Behandlung von biologischem Material mittels elektrischen Stroms, bei dem das biologische Material in einer Pufferlösung mit einer lonenstärke von mindestens 100 mmol/l aufgenommen und in der Pufferlösung durch zumindest einen Spannungsimpuls ein elektrisches Feld mit einer Feldstärke von mindestens 1 kV/cm für eine voreingestellte Dauer von mindestens 10 µs erzeugt wird, **dadurch gekennzeichnet, dass** die Pufferlösung ein Volumen von höchstens 50 µl hat und der Spannungsimpuls mindestens einmal für eine Zeitspanne von mindestens 100 µs unterbrochen und anschließend wieder fortgesetzt wird, wobei das Unterbrechen und Fortsetzen des Spannungsimpulses so oft ausgeführt wird, bis die voreingestellte Dauer des Spannungsimpulses erreicht ist.

2. Verfahren nach Anspruch 1, **dadurch gekennzeichnet, dass** der Spannungsimpuls zwei- bis zehnmal unterbrochen wird.

3. Verfahren nach Anspruch 1 oder 2, **dadurch gekennzeichnet, dass** für zumindest eine Unterbrechung eine Zeitspanne von 200 µs bis 2 ms, vorzugsweise 300 µs, 400 µs, 500 µs, 600 µs, 700 µs, 800 µs, 900 µs, 1 ms oder 1,5 ms, vorgegeben wird.

4. Verfahren nach einem der Ansprüche 1 bis 3, **dadurch gekennzeichnet, dass** das Volumen der Pufferlösung mit dem biologischen Material insgesamt zwischen 1 und 50 µl, vorzugsweise 10 und 40 µl, besonders bevorzugt 15 und 25 µl, insbesondere bei 10 bis 20 µl, liegt.

5. Verfahren nach einem der Ansprüche 1 bis 4, **dadurch gekennzeichnet, dass** der Spannungsimpuls ein elektrisches Feld mit einer Feldstärke von maximal 10 kV/cm, vorzugsweise 1 bis 8 kV/cm, besonders bevorzugt 2 bis 6 kV/cm, insbesondere 2 bis 4 kV/cm, erzeugt.

6. Verfahren nach einem der Ansprüche 1 bis 5, **dadurch gekennzeichnet, dass** der Spannungsimpuls eine voreingestellte Dauer von maximal 5 ms, vorzugsweise 20 µs bis 2 ms, besonders bevorzugt 100 bis 1000 µs, insbesondere 100 bis 600 µs, aufweist.

7. Verfahren nach einem der Ansprüche 1 bis 6, **dadurch gekennzeichnet, dass** der Spannungsimpuls annähernd nach einem Spannungsintervall von 5 µs, 10 µs, 20 µs, 30 µs, 40 µs, 50 µs, 60 µs, 100 µs oder 200 µs unterbrochen wird.

8. Verfahren nach einem der Ansprüche 1 bis 7, **dadurch gekennzeichnet, dass** das elektrische Feld zwischen zwei Elektroden erzeugt wird und der Abstand zwischen den beiden Elektroden 0,5 bis 5 mm, vorzugsweise 1 bis 4 mm, insbesondere 1,5 bis 2 mm, beträgt.

9. Verfahren nach einem der Ansprüche 1 bis 8, **dadurch gekennzeichnet, dass** die Behandlung des biologischen Materials in einem Reaktionsbehältnis durchgeführt wird, das einen im Wesentlichen quadratischen, rechteckigen oder runden Querschnitt hat.

10. Verfahren nach Anspruch 9, **dadurch gekennzeichnet, dass** das Reaktionsbehältnis einen im wesentlichen rechteckigen Reaktionsraum aufweist, der seitlich durch zwei planparallel angeordnete Elektroden begrenzt ist.

## Claims

1. A method for treating biological material by means of electrical current, wherein the biological material is added to the buffer solution which has an ionic strength of at least 100 mmol/l, and at least one voltage pulse is applied to said buffer solution for a preset duration of at least 10 µs to generate an electrical field having a field strength of at least 1 kV/cm, **characterized in that** the buffer solution has a volume of at most 50 µl, and wherein the voltage pulse is interrupted at least once for a duration of at least 100 µs and is subsequently continued, wherein the interruption and subsequent continuation of the voltage pulse is repeated as often as required to reach the preset duration of the voltage pulse.

2. The method according to claim 1, **characterized in that** the voltage pulse is interrupted twice to ten times.

3. The method according to claim 1 or 2, **characterized in that** a duration of 200 µs to 2 ms, preferably 300 µs, 400 µs, 500 µs, 600 µs, 700 µs, 800 µs, 900 µs, 1 ms or 1.5 ms is preset for at least one interruption.

4. The method according to any one of claims 1 to 3, **characterized in that** the buffer solution including the biological material has a total volume of between 1 and 50 µl, preferably 10 and 40 µl, particularly preferably 15 and 25 µl, in particular 10 to 20 µl.

5. The method according to any one of claims 1 to 4, **characterized in that** the voltage pulse generates an electrical field with a field strength having a maximum of 10 kV/cm, preferably 1 to 8 kV/cm, particularly preferably 2 to 6 kV/cm, in particular 2 to 4 kV/cm.

6. The method according to any one of claims 1 to 5, **characterized in that** the voltage pulse has a preset duration of a maximum of 5 ms, preferably 20 µs to 2 ms, particularly preferably 100 to 1000 µs, in particular 100 to 600 µs.

7. The method according to any one of claims 1 to 6, **characterized in that** the voltage pulse is interrupted approximately after a voltage interval of 5 µs, 10 µs, 20 µs, 30 µs, 40 µs, 50 µs, 60 µs, 100 µs or 200 µs.

8. The method according to any one of claims 1 to 7, **characterized in that** the electrical field is generated between two electrodes and **in that** the distance between the two electrodes is 0.5 to 5 mm, preferably 1 to 4 mm, in particular 1.5 to 2 mm.

9. The method according to any one of claims 1 to 8, **characterized in that** the biological material is treated in a reaction container which has a substantially square, rectangular or round cross-section.

10. The method according to claim 9, **characterized in that** the reaction container has a substantially rectangular reaction chamber which is delimited laterally by two electrodes in a plane-parallel configuration.

## Revendications

1. Procédé pour le traitement de matériau biologique au moyen de courant électrique, dans lequel le matériau biologique est absorbé dans une solution tampon présentant une force ionique d'au moins 100 mmol/l et un champ électrique présentant une intensité de champ d'au moins 1 kV/cm pour une durée préréglée d'au moins 10 µs est générée dans la solution tampon par au moins une impulsion de tension, **caractérisé en ce que** la solution tampon a un volume maximum de 50 µl et l'impulsion de tension est interrompue au moins une fois pour un laps de temps d'au moins 100 µs et est poursuivie à nouveau ensuite, l'interruption et la poursuite de l'impulsion de tension étant effectuées à chaque fois jusqu'à ce que la durée préréglée de l'impulsion de tension soit atteinte.

2. Procédé selon la revendication 1, **caractérisé en ce que** l'impulsion de tension est interrompue deux fois à dix fois.

3. Procédé selon la revendication 1 ou 2, **caractérisé en ce que**, pour au moins une interruption, un laps de temps de 200 µs à 2 ms, de préférence 300 µs, 400 µs, 500 µs, 600 µs, 700 µs, 800 µs, 900 µs, 1 ms ou 1,5 ms est prédéfini.

4. Procédé selon l'une quelconque des revendications 1 à 3, **caractérisé en ce que** la zone de la solution tampon avec le matériau biologique se situe globalement entre 1 et 50 µl, de préférence 10 et 40 µl, avec une préférence particulière entre 15 et 25 µl, en particulier entre 10 et 20 µl.

5. Procédé selon l'une quelconque des revendications 1 à 4, **caractérisé en ce que** l'impulsion de tension génère un champ électrique avec une intensité de champ de 10 kV/cm, de préférence 1 à 8 kV/cm, avec une préférence particulière 2 jusqu'à 6 kV/cm, en particulier 2 à 4 kV/cm.

6. Procédé selon l'une quelconque des revendications 1 à 5, **caractérisé en ce que** l'impulsion de tension présente une durée préréglée maximale de 5 ms, de préférence de 20 µs à 2 ms, avec une préférence particulière 100 à 1000 µs, en particulier 100 à 600 µs.

7. Procédé selon l'une quelconque des revendications 1 à 6, **caractérisé en ce que** l'impulsion de tension est interrompue approximativement après un intervalle de tension de 5 µs 10 µs, 20 µs, 30 µs, 40 µs, 50 µs, 60 µs, 100 µs ou 200 µs.

8. Procédé selon l'une quelconque des revendications 1 à 7, **caractérisé en ce que** le champ électrique est généré entre deux électrodes et la distance entre les deux électrodes est de 0,5 à 5 mm, de préférence 1 à 4 mm, en particulier 1,5 à 2 mm.

9. Procédé selon l'une quelconque des revendications 1 à 8, **caractérisé en ce que** le traitement du matériau biologique est effectué dans un récipient de réaction, qui présente une section sensiblement carrée, rectangulaire ou ronde.

10. Procédé selon la revendication 9, **caractérisé en ce que** le récipient de réaction présente un espace de réaction sensiblement rectangulaire, qui est limité sur le côté par deux électrodes disposées planes et parallèles.
